# EUROPEAN PATENT SPECIFICATION

(11) **EP 1 676 134 B1**
(45) Date of publication and mention of the grant of the patent: **23.04.2008**
(21) Application number: 04790449.5
(22) Date of filing: 15.10.2004
(51) Int. Cl.: G01N 33/574

(54) **USE OF PROTEIN SPEE AS A MARKER FOR BREAST CANCER**
VERWENDUNG DES PROTEINS SPEE ALS MARKER FÜR BRUSTKREBS
UTILISATION D'UNE PROTEINE SPEE (SPERMIDINE SYNTHASE) EN TANT QUE MARQUEUR DU CANCER DU SEIN

(30) Priority: 15.10.2003 EP 03023508
(43) Date of publication of application: 05.07.2006
(73) Proprietor: Roche Diagnostics GmbH, 68305 Mannheim (DE); F. Hoffmann-La Roche AG, 4070 Basel (CH)
(72) Inventor: PESTLIN, Gabriele, 81679 Muenchen (DE); BERNDT, Peter, CH-4056 Basel (DE); HAGMANN, Marie-Luise, 82377 Penzberg (DE); KARL, Johann, 82380 Peissenberg (DE); LANGEN, Hanno, 79540 Lörrach (DE); WILD, Norbert, 82538 Geretsried (DE); ZOLG, Werner, 82362 Weilheim (DE)
(86) International application number: PCT/EP2004/011595
(87) International publication number: WO 2005/040804

(56) References cited:
- WO-A-02/46471
- WO-A-02/059377
- WO-A-03/075945
- SHIRAHATA A ET AL: "Monospecific antiserum to rat spermidine synthase and its application to rat tissues and several mammals." JOURNAL OF BIOCHEMISTRY. NOV 1988, vol. 104, no. 5, November 1988 (1988-11), pages 717-721, XP008043144 ISSN: 0021-924X
- MANNI A: "POLYAMINES AND HORMONAL CONTROL OF BREAST CANCER GROWTH" CRITICAL REVIEWS IN ONCOGENESIS, vol. 1, no. 2, 1989, pages 163-174, XP008043073 ISSN: 0893-9675
- LIPTON A ET AL: "URINARY POLY AMINE LEVELS IN HUMAN CANCER" CANCER, vol. 35, no. 2, 1975, pages 464-468, XP008043151 ISSN: 0008-543X
- HORN Y ET AL: "FUTHER EVIDENCE FOR THE USE OF POLY AMINES AS BIOCHEMICAL MARKERS FOR MALIGNANT TUMORS" CANCER RESEARCH, vol. 42, no. 8, 1982, pages 3248-3251, XP008043149 ISSN: 0008-5472

### SEQUENCE LISTING

<110> Roche Diagnostics GmbH F. Hoffmann-La Roche AG
<120> Use of protein SPEE as a marker for breast cancer
<130> 22239
<150> EP 03023508.9
   <151> 2003-10-15
<160> 1
<170> PatentIn version 3.2
<210> 1
   <211> 302
   <212> PRT
   <213> Homo sapiens
<220>
   <221> MISC_FEATURE
   <223> spermidine synthase (SPEE), also known as putrescine aminopropyltransferase
<400> 1
   Met Glu Pro Gly Pro Asp Gly Pro Ala Ala Ser Gly Pro Ala Ala Ile
   1               5                   10                  15
   Arg Glu Gly Trp Phe Arg Glu Thr Cys Ser Leu Trp Pro Gly Gln Ala
               20                  25                  30
   Leu Ser Leu Gln Val Glu Gln Leu Leu His His Arg Arg Ser Arg Tyr
           35                  40                  45
   Gln Asp Ile Leu Val Phe Arg Ser Lys Thr Tyr Gly Asn Val Leu Val
       50                  55                  60
   Leu Asp Gly Val Ile Gln Cys Thr Glu Arg Asp Glu Phe Ser Tyr Gln
   65                  70                  75                  80
   Glu Met Ile Ala Asn Leu Pro Leu Cys Ser His Pro Asn Pro Arg Lys
                   85                  90                  95
   Val Leu Ile Ile Gly Gly Gly Asp Gly Gly Val Leu Arg Glu Val Val
               100                 105                 110
   Lys His Pro Ser Val Glu Ser Val Val Gln Cys Glu Ile Asp Glu Asp
   115 120 125
   Val Ile Gln Val Ser Lys Lys Phe Leu Pro Gly Met Ala Ile Gly Tyr
   130 135 140
   Ser Ser Ser Lys Leu Thr Leu His Val Gly Asp Gly Phe Glu Phe Met
   145 150 155 160
   Lys Gln Asn Gln Asp Ala Phe Asp Val Ile Ile Thr Asp Ser Ser Asp
   165 170 175
   Pro Met Gly Pro Ala Glu Ser Leu Phe Lys Glu Ser Tyr Tyr Gln Leu
   180 185 190
   Met Lys Thr Ala Leu Lys Glu Asp Gly Val Leu Cys Cys Gln Gly Glu
   195 200 205
   Cys Gln Trp Leu His Leu Asp Leu Ile Lys Glu Met Arg Gln Phe Cys
   210 215 220
   Gln Ser Leu Phe Pro Val Val Ala Tyr Ala Tyr Cys Thr Ile Pro Thr
   225 230 235 240
   Tyr Pro Ser Gly Gln Ile Gly Phe Met Leu Cys Ser Lys Asn Pro Ser
   245 250 255
   Thr Asn Phe Gln Glu Pro Val Gln Pro Leu Thr Gln Gln Gln Val Ala
   260 265 270
   Gln Met Gln Leu Lys Tyr Tyr Asn Ser Asp Val His Arg Ala Ala Phe
   275 280 285
   Val Leu Pro Glu Phe Ala Arg Lys Ala Leu Asn Asp Val Ser
   290 295 300

## Description

The present invention relates to the diagnosis of breast cancer. It discloses the use of spermidine synthase (= SPEE) in the diagnosis of breast cancer. Furthermore, it especially relates to a method for diagnosis of breast cancer from a liquid sample, derived from an individual by measuring SPEE in said sample. Measurement of SPEE can, e.g., be used in the early detection or diagnosis of breast cancer.

Cancer remains a major public health challenge despite progress in detection and therapy. Amongst the various types of cancer, breast cancer (=BC) is one of the most frequent cancers among women in the Western world.

The earlier cancer can be detected/diagnosed, the better is the overall survival rate. This is especially true for BC. The prognosis in advanced stages of tumor is poor. More than one third of the patients will die from progressive disease within five years after diagnosis, corresponding to a survival rate of about 40% for five years. Current treatment is only curing a fraction of the patients and clearly has the best effect on those patients diagnosed in an early stage of disease.

With regard to BC as a public health problem, it is essential that more effective screening and preventative measures for breast cancer will be developed.

The earliest detection procedures available at present for breast cancer involve using clinical breast examination and mammography. However, significant tumor size must typically exist before a tumor is palpable or can be detected by a mammogram. The densitiy of the breast tissue and the age are important predictors of the accuracy of screening mammography. The sensitivity ranges from 63 % in women with extremely dense breasts to 87 % in women with almost entirely fatty breasts. The sensitivity increases with age from 69 % in women of about 40 years of age to 83 % in women 80 years and older (Carney, P.A., et al., Ann. Intern. Med. 138 (3) (2003) 168-175). Only 20 - 25 % of mammographically detected abnormalities that are biopsied prove to be malignant. The visualization of precancerous and cancerous lesions represents the best approach to early detection, but mammography is an expensive test that requires great care and expertise both to perform and in the interpretation of results (WHO, Screening for Breast Cancer, May 10, 2002; Esserman, L., et al., J. Natl. Cancer Inst. 94 (2002) 369-375).

In the recent years a tremendous amount of so-called breast specific or even so-called breast cancer specific genes has been reported. The vast majority of the corresponding research papers or patent applications are based on data obtained by analysis of RNA expression patterns in breast (cancer) tissue versus a different tissue or an adjacent normal tissue, respectively. Such approaches may be summarized as differential mRNA display techniques.

As an example for data available from mRNA-display techniques, WO 00/60076 shall be mentioned and discussed. This application describes and claims more than two hundred isolated polynucleotides and the corresponding polypeptides as such, as well as their use in the detection of BC. However, it is general knowledge that differences on the level of mRNA are not mirrored by the level of the corresponding proteins. A protein encoded by a rare mRNA may be found in very high amounts and a protein encoded by an abundant mRNA may nonetheless be hard to detect and find at all (Chen, G., et al., Molecular and Cellular Proteomics, 1.4 (2002) 304-313). This lack of correlation between mRNA-level and protein level is due to reasons like mRNA stability, efficiency of translation, stability of the protein, etc.

There also are recent approaches investigating the differences in protein patterns between different tissues or between healthy and diseased tissue in order to identify candidate marker molecules which might be used in the diagnosis of BC. Wulfkuhle et al. Cancer Research 62 (2002) 6740-6749 have identified fifty-seven proteins which were differentially expressed between BC tissue and adjacent normal tissue. No data from liquid samples obtained from an individual are reported.

WO 02/23200 reports about twelve breast cancer-associated spots as found by surface-enhanced laser desorption and ionization (SELDI). These spots are seen more frequently in sera obtained from patients with BC as compared to sera obtained from healthy controls. However, the identity of the molecule(s) comprised in such spot, e.g their sequence, is not known.

Nipple aspirate fluid (NAF) has been used for many years as a potential non-invasive method to identify breast cancer-specific markers. Kuerer et al. compared bilateral matched pair nipple aspirate fluids from women with unilateral invasive breast carcinoma by 2D gel electrophoresis (Kuerer, H.M., et al., Cancer 95 (2002) 2276-2282). 30 to 202 different protein spots were detected in the NAF of breasts suffering from breast carcinoma and not in the matched NAF of the healthy breasts. These spots were detected by a gel image analysis. But the identity of the protein spots is not known.

Despite the large and ever growing list of candidate protein markers in the field of BC, to date clinical/diagnostic utility of these molecules is not known. In order to be of clinical utility a new diagnostic marker as a single marker should be at least as good as the best single marker known in the art. Or, a new marker should lead to a progress in diagnostic sensitivity and/or specificity either if used alone or in combination with one or more other markers, respectively. The diagnostic sensitivity and/or specificity of a test is best assessed by its receiver-operating characteristics, which will be described in detail below.

At present, only diagnostic blood tests based on the detection of cancer antigen 15-3 (CA 15-3), a tumor-associated mucin, and carcinoembryonic antigen (CEA), a tumor associated glycoprotein, are available to assist diagnosis in the field of BC. CA 15-3 is usually increased in patients with advanced breast cancer. CA 15-3 levels are rarely elevated in women with early stage breast cancer (Duffy, M.J., Critical Reviews in Clinical Laboratory Sciences 38 (2001) 225-262). Cancers of the ovary, lung and prostate may also raise CA 15-3 levels. Elevated levels of CA 15-3 may be associated with non-cancerous conditions, such as benign breast or ovary disease, endometriosis, pelvic inflammatory disease, and hepatitis. Pregnancy and lactation can also cause CA 15-3 levels to raise (National Cancer Institute, Cancer Facts, Fact Sheet 5.18 (1998) 1-5). The primary use of CEA is in monitoring colon cancer, especially when the disease has metastasized. However, a variety of cancers can produce elevated levels of CEA, including breast cancer.

Due to the lack of organ and tumor specificity, neither measurement of CA 15-3 nor measurement of CEA are recommended for screening of BC. These tumor markers are helpful diagnostic tools in follow-up care of BC patients (Untch, M., et al., J. Lab. Med. 25 (2001) 343-352).

Whole blood, serum, plasma, or nipple aspirate fluid are the most widely used sources of sample in clinical routine. The identification of an early BC tumor marker that would allow reliable cancer detection or provide early prognostic information could lead to a diagnostic assay that would greatly aid in the diagnosis and in the management of this disease. Therefore, an urgent clinical need exists to improve the diagnosis of BC from blood. It is especially important to improve the early diagnosis of BC, since for patients diagnosed early on chances of survival are much higher as compared to those diagnosed at a progressed stage of disease.

It was the task of the present invention to investigate whether a new marker can be identified which may aid in BC diagnosis.

Surprisingly, it has been found that use of the marker SPEE can at least partially overcome the problems known from the state of the art.

The present invention therefore relates to a method for the diagnosis of breast cancer comprising the steps of a) providing a liquid sample obtained from an individual, b) contacting said sample with a specific binding agent for SPEE under conditions appropriate for formation of a complex between said binding agent and SPEE, and c) correlating the amount of complex formed in (b) to the diagnosis of breast cancer.

Another preferred embodiment of the invention is a method for the diagnosis of breast cancer comprising the steps of a) contacting a liquid sample obtained from an individual with a specific binding agent for-SPEE under conditions appropriate for formation of a complex between said binding agent and SPEE, and b) correlating the amount of complex formed in (a) to the diagnosis of breast cancer.

As the skilled artisan will appreciate, any such diagnosis is made in vitro. The patient sample is discarded afterwards. The patient sample is merely used for the in vitro diagnostic method of the invention and the material of the patient sample is not transferred back into the patient's body. Typically, the sample is a liquid sample.

The spermidine synthase (SPEE; also known as putrescine aminopropyltransferase; Swiss-PROT: P19623) is characterized by the sequence given in SEQ ID NO:1. This sequence translates to a theoretical molecular weight of 33824 Da and to an isoelectric point at pH 5.26.

The biosynthesis of polyamines, which are essential for cellular functions, involves four distinct enzymes: ornithine decarboxylase, S-adenosyl-L-methionine decarboxylase, spermine synthase, and spermidine synthase. Wahlfors et al. cloned a cDNA coding for the full-length subunit of spermidine synthase (Wahlfors, J., et al., DNA Cell Biol. 9 (1990) 103-110).

The-biosynthesis of the polyamine spermidine is catalyzed by the spermidine synthase which transfers an aminopropyl moiety from decarboxylated S-adenosyl methionine to putrescine. Spermidine binds to DNA and is implicated in a number of crucial processes such as cell division, differentiation, and membrane function. The inhibition of polyamine biosynthesis stops cell growth (Kaiser, A.E., et al., Folia Parasitologica 50 (2003) 3-18).

The catalytic activity of spermidine synthase can be inhibited by methylthiopropylamine (MTPA). Hibasami et al. showed that the inhibition of spermidine synthase lead to inhibited growth of human lymphoid leukemia Molt 4B cells (Hibasami, H., et al., Anticancer Research 7 (1987) 1213-1216).

Nishikawa et al. found out that the spermidine synthase mRNA expression and its enzyme activity were decreased after treatment of hepatoma cell-lines Hep 3B with the protein transforming growth factor beta (TGF-betal). They suggested that the down-regulation of spermidine synthase may be associated with the mechanism of TGF-beta-induced growth suppression (Nishikawa, Y., et al., Biochem. J. 321 (1997) 537-543).

Nitta et al. investigated the effect of intracellular polyamine depletion on apoptosis. They found out that the inhibition of spermidine synthase and similar enzymes and the resulting decrease of polyamines trigger the mitochondria-mediated pathway for apoptosis, resulting in caspase activation and apoptotic cell death (Nitta, T., et al., Exp. Cell Res. 276 (2002) 120-128).

Spermidine synthase has been mentioned in the patent application WO 02/059377 besides a large number of genes and their proteins for diagnosing breast cancer. But the diagnostic application has not been described.

WO 02/046471 is concerned with the identification of markers that can be used to determine whether cancer cells are sensitive to a therapeutic agent. The level of SPEE-mRNA alike the level of mRNAs from many other genes is mentioned to be modulated by an anti-cancer drug.

As obvious to the skilled artisan, the present invention shall not be construed to be limited to the full-length protein SPEE of SEQ ID NO:1. Physiological or artificial fragments of SPEE, secondary modifications of SPEE, as well as allelic variants of SPEE are also encompassed by the present invention. Artificial fragments preferably encompass a peptide produced synthetically or by recombinant techniques, which at least comprises one epitope of diagnostic interest consisting of at least 6 contiguous amino acids as derived from the sequence disclosed in SEQ ID NO:1. Such fragment may advantageously be used for generation of antibodies or as a standard in an immunoassay. More preferred the artificial fragment comprises at least two epitopes of interest appropriate for setting up a sandwich immunoassay.

In preferred embodiments, the novel marker SPEE may be used for monitoring as well as for screening purposes.

When used in patient monitoring the diagnostic method according to the present invention may help to assess tumor load, efficacy of treatment and tumor recurrence in the follow-up of patients. Increased levels of SPEE are directly correlated to tumor burden. After chemotherapy a short term (few hours to 14 days) increase in SPEE may serve as an indicator of tumor cell death. In the follow-up of patients (from 3 months to 10 years) an increase of SPEE can be used as an indicator for tumor recurrence.

In a preferred embodiment the diagnostic method according to the present invention is used for screening purposes. I.e., it is used to assess subjects without a prior diagnosis of BC by measuring the level of SPEE and correlating the level measured to the presence or absence of BC.

The staging of cancer is the classification of the disease in terms of extent, progression, and severity. It groups cancer patients so that generalizations can be made about prognosis and the choice of therapy.

Today, the TNM system is the most widely used classification of the anatomical extent of cancer. It represents an internationally accepted, uniform staging system. There are three basic variables: T (the extent of the primary tumor), N (the status of regional lymph nodes) and M (the presence or absence of distant metastases). The TNM criteria are published by the UICC (International Union Against Cancer) (Sobin, L.H., Wittekind, Ch. (eds): TNM Classification of Malignant Tumours, fifth edition, 1997). The staging system for breast cancer has recently been revised (Singletary, S.E., et al., Journal of Clinical Oncology 20 (2002) 3628-3636).

What is especially important is, that early diagnosis of BC translates to a much better prognosis. Therefore, best prognosis have those patients as early as in stage Tᵢₛ, N0, M0 or T1-3; N0; M0, if treated properly have a more than 90% chance of survival 5 years after diagnosis as compared to a 5-years survival rate of only 18% for patients diagnosed when distant metastases are already present.

In the sense of the present invention early diagnosis of BC refers to a diagnosis at a pre-cancerous state (DCIS) or at a tumor stage where no metastases at all (neither proximal nor distal), i.e., Tᵢₛ, N0, M0 or T1-4; N0; M0 are present. Tᵢₛ denotes carcinoma *in situ.*

In a preferred embodiment SPEE is used to diagnose BC in a non-metastatic stage, i.e., that diagnosis is made at stage Tᵢₛ, N0, M0 or T1-3; N0; M0 (=Tᵢₛ-3; N0; M0).

The diagnostic method according to the present invention is based on a liquid sample which is derived from an individual. Unlike to methods known from the art SPEE is specifically measured from this liquid sample by use of a specific binding agent.

A specific binding agent is, e.g., a receptor for SPEE, a lectin binding to SPEE or an antibody to SPEE. A specific binding agent has at least an affinity of 10⁷ l/mol for its corresponding target molecule. The specific binding agent preferably has an affinity of 10⁸ 1/mol or even more preferred of 10⁹ l/mol for its target molecule. As the skilled artisan will appreciate the term specific is used to indicate that other biomolecules present in the sample do not significantly bind to with the binding agent specific for SPEE. Preferably, the level of binding to a biomolecule other than the target molecule results in a binding affinity which is only 10%, more preferably only 5% of the affinity of the target molecule or less. A most preferred specific binding agent will fulfill both the above minimum criteria for affinity as well as for specificity.

A specific binding agent preferably is an antibody reactive with SPEE.. The term antibody refers to a polyclonal antibody, a monoclonal antibody, fragments of such antibodies, as well as to genetic constructs comprising the binding domain of an antibody. Any antibody fragment retaining the above criteria of a specific binding agent can also be used.

Antibodies are generated by state of the art procedures, e.g., as described in Tijssen (Tijssen, P., Practice and theory of enzyme immunoassays 11 (1990) the whole book, especially pages 43-78; Elsevier, Amsterdam). In addition, the skilled artisan is well aware of methods based on immunosorbents that can be used for the specific isolation of antibodies. By these means the quality of polyclonal antibodies and hence their performance in immunoassays can be enhanced. (Tijssen, P., *supra,* pages 108-115).

For the achievements as disclosed in the present invention monoclonal and polyclonal antibodies have been used. Polyclonal antibodies have been raised in rabbits. However, clearly also polyclonal antibodies from different species , e.g. rats or guinea pigs can also be used. Monoclonal antibodies have been produced using spleen cells from immunized mice. Since monoclonal antibodies can be produced in any amount required with constant properties, they represent ideal tools in development of an assay for clinical routine. The use of monoclonal antibodies to SPEE in a method according to the present invention is yet another

### preferred embodiment.

As the skilled artisan will appreciate, various strategies may be used to generate antibodies to SPEE. Such strategies comprise amongst others the use of synthetic peptides, representing an epitope of SPEE for immunization. Preferably, a synthetic peptide comprises a subsequence of SEQ ID NO:1 which is specific for SPEE, i.e., which has a comparatively low homology to other/related polypeptides. It is preferred that the synthetic peptide comprises a contiguous subsequence consisting of 5 to 25 amino acid residues of SEQ ID NO:1. More preferred, the peptide comprises a contiguous subsequence consisting of 10 to 15 amino acid residues of SEQ ID NO:1.

Alternatively, DNA immunization also known as DNA vaccination may be used.

For measurement the liquid sample obtained from an individual is incubated with the specific binding agent for SPEE under conditions appropriate for formation of a binding agent SPEE-complex. Such conditions need not be specified, since the skilled artisan without any inventive effort can easily identify such appropriate incubation conditions.

As a final step according to the method disclosed in the present invention the amount of complex is measured and correlated to the diagnosis of BC. As the skilled artisan will appreciate there are numerous methods to measure the amount of specific binding agent SPEE-complex all described in detail in relevant textbooks (cf., e.g., Tijssen P., supra, or Diamandis et al., eds. (1996) Immunoassay, Academic Press, Boston).

Preferably SPEE is detected in a sandwich type assay format. In such assay a first specific binding agent is used to capture SPEE on the one side and a second specific binding agent, which is labeled to be directly or indirectly detectable is used on the other side.

As mentioned above, it has surprisingly been found that SPEE can be measured from a liquid sample obtained from an individual sample. No tissue and no biopsy sample is required to apply the marker SPEE in the diagnosis of BC.

In a preferred embodiment the method according to the present invention is practiced with serum as liquid sample material.

In a further preferred embodiment the method according to the present invention is practiced with plasma as liquid sample material.

In a further preferred embodiment the method according to the present invention is practiced with whole blood as liquid sample material.

In a further preferred embodiment the method according to the present invention is practiced with nipple aspirate fluid as liquid sample material.

Whereas application of routine proteomics methods to tissue samples, leads to the identification of many potential marker candidates for the tissue selected, the inventors of the present invention have surprisingly been able to detect SPEE in a bodily fluid sample. Even more surprising they have been able to demonstrate that the presence of SPEE in such liquid sample obtained from an individual can be correlated to the diagnosis of breast cancer.

Antibodies to SPEE with great advantage can be used in established procedures, e.g., to detect breast cancer cells in situ, in biopsies, or in immunohistological procedures.

Preferably, an antibody to SPEE is used in a qualitative (SPEE present or absent) or quantitative (SPEE amount is determined) immunoassay.

Measuring the level of protein SPEE has proven very advantageous in the field of BC. Therefore, in a further preferred embodiment, the present invention relates to use of protein SPEE as a marker molecule in the diagnosis of breast cancer from a liquid sample obtained from an individual.

The term marker molecule is used to indicate that an increased level of the analyte SPEE as measured from a bodily fluid of an individual-marks the presence of BC.

It is especially preferred to use the novel marker SPEE in the early diagnosis of breast cancer.

The use of protein SPEE itself, represents a significant progress to the challenging -field- of BC diagnosis. Combining measurements of SPEE with other known markers, e.g. CA 15-3 and CEA, or with other markers of BC presently known or yet to be discovered, leads to further improvements. Therefore in a further preferred embodiment the present invention relates to the use of SPEE as a marker molecule for breast cancer in combination with one or more marker molecules for breast cancer in the diagnosis of breast cancer from a liquid sample obtained from an individual. In this regard, the expression "one or more" denotes 1 to 10, preferably 1 to 5, more preferred 3. Preferred selected other BC markers with which the measurement of SPEE may be combined are CEA and CA 15-3. Most preferred, SPEE is used as part of a marker panel at least comprising SPEE and CA 15-3. Thus, a further preferred embodiment of the present invention is the use of the protein SPEE as a marker molecule for breast cancer in combination with one or more marker molecules for breast cancer in the diagnosis of breast cancer from a liquid sample obtained from an individual, whereby the at least one other marker molecule is CA 15-3.

Preferably, the inventive method is used with samples of patients suspected of suffering from breast cancer. An individual suspected of suffering from breast cancer is an individual for which other types of cancers have been excluded. Other cancers include but are not limited to cancers of the colon, lung, stomach, ovary, and prostate. A preferred embodiment of the invention is therefore a method for the diagnosis of breast cancer comprising the steps of a) providing a liquid sample obtained from an individual suspected of suffering from breast cancer, b) contacting said sample with a specific binding agent for SPEE under conditions appropriate for formation of a complex between said binding agent and SPEE, and c) correlating the amount of complex formed in (b) to the diagnosis of breast cancer.

Diagnostic reagents in the field of specific binding assays, like immunoassays, usually are best provided in the form of a kit, which comprises the specific binding agent and the auxiliary reagents required to perform the assay.

Accuracy of a test is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results) (number of true-positive + number of false-negative test results)]. This has also been referred to as positivity in the presence of a disease or condition. It is calculated solely from the affected subgroup. On the x-axis is the false-positive fraction, or 1 specificity [defined as (number of false-positive results) / (number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion- for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Clinical utility of the novel marker SPEE has been assessed in comparison to and in combination with the established marker CA 15-3 using a receiver operator curve analysis (ROC; Zweig, M. H., and Campbell, G., Clin. Chem. 39 (1993) 561-577). This analysis has been based on well-defined patient cohorts consisting of 50 samples each from patients with invasive ductal or lobular carcinoma in T1-3; N0; M0, more progressed tumor, i.e., T4 and/or various severity of metastasis (N+ and/or M+), medullary, papillary, mucinous and tubular carcinoma, ductal carcinoma in situ, and healthy controls, respectively.

The following examples, references, sequence listing and figure are provided to aid the understanding of the present invention, the true scope of which is set forth in the appended claims.

### Description of the Figure

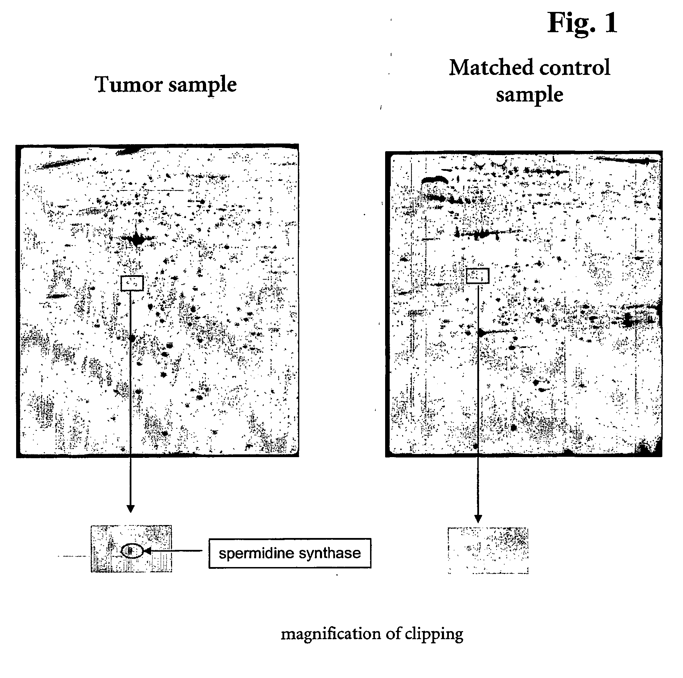

- Figure 1: Figure 1 shows a typical example of a 2D-gel, loaded with a tumor sample (left side), and a gel, loaded with a matched control sample (right side). The circle in the enlarged section of these gels indicates the position for the protein SPEE. Using the same method this protein has not been detected in healthy tissue. SPEE migrated in the 2D gel corresponding to an isoelectric point of about pH 5.3 and an apparent molecular weight of between 30 and 35 kDa.

### Abbreviations

- ABTS: 2,2'-Azino-di- [3-ethylbenzthiazoline sulfonate (6)] diammonium salt
- BSA: bovine serum albumin
- cDNA: complementary DNA
- CHAPS: (3-[(3-Cholamidopropyl)-dimethylammonio]-1-propane-sulfonate)
- DMSO: dimethyl sulfoxide
- DTT: dithiothreitol
- EDTA: ethylene diamine tetraacetic acid
- ELISA: enzyme-linked immunosorbent assay
- HRP: horseradish peroxidase
- IAA: iodacetamid
- IgG: immunoglobulin G
- IEF: isoelectric focussing
- IPG: immobilized pH gradient
- LDS: lithium dodecyl sulfate
- MALDI-TOF: matrix-assisted laser desorption/ionisation-time of flight mass spectrometry
- MES: mesityl, 2,4,6-trimethylphenyl
- OD: optical density
- PAGE: polyacrylamide gel electrophoresis
- PBS: phosphate buffered saline
- PI: isoelectric point
- RTS: rapid translation system
- SDS: sodium dodecyl sulfate
- UICC: International Union Against Cancer

### Example 1

### Identification of SPEE as a potential breast cancer marker

### Sources of tissue

In order to identify tumor-specific proteins as potential diagnostic markers for breast cancer, analysis of two different kinds of tissue is performed using proteomics methods.

In total, tissue specimen from 14 patients suffering from breast cancer are analyzed. From each patient two different tissue types are collected from therapeutic resections: Tumor tissue (> 80% tumor) (T), and adjacent healthy tissue (N). The latter tissue type serves as matched healthy control sample. Tissues are immediately snap frozen after resection and stored at -80°C before processing. Tumors are diagnosed by histopathological criteria.

### Tissue preparation

0.8-1.2 g of frozen tissue are put into a mortar and completely frozen by liquid nitrogen. The tissue is pulverized in the mortar, dissolved in the 10-fold volume (w/v) of lysis buffer (40 mM Na-citrate, 5 mM MgCl₂, 1% Genapol X-080, 0.02% Na-azide, Complete^{®} EDTA-free [Roche Diagnostics GmbH, Mannheim, Germany, Cat. No. 1 873 580]) and subsequently homogenized in a Wheaton^{®} glass homogenizer (20 x loose fitting, 20 x tight fitting). 3 ml of the homogenate are subjected to a sucrose-density centrifugation (10-60% sucrose) for 1 h at 4,500 x g. After this centrifugation step three fractions are obtained. The fraction on top of the gradient contains the soluble proteins and is used for further analysis.

### Immobilization of monoclonal antibody anti-human albumin on CNBr-activated Sepharose 4B

Freeze-dried CNBr-activated Sepharose 4B (Amersham Biosciences, 17-0430-01) is reswollen and washed according to the instructions of the manufacturer. Monoclonal antibody directed against human albumin is dissolved in 0.1 M NaHCO₃, pH 8.3, 0.5 M NaCl, 10 mg/ml. 1 ml antibody solution is mixed with 1 ml reswollen CNBr-activated Sepharose 4B. The reaction time is 1 h. Blocking of the remaining acitve groups and washing of the gel is carried out according to the instructions of the manufacturer.

### Depletion of serum albumin

7 ml anti-albumin gel is equilibrated in lysis buffer without Genapol X-080. 7 ml of the upper fraction of the sucrose-density centrifugation (see above, tissue preparation) are applied onto the column and washed through with lysis buffer without Genalpol X-080. The combined effluent is used for the isoelectric focussing experiments.

### Isoelectric focussing (IEF) and SDS-PAGE

For IEF, 3 ml of the HSA-depleted tissue preparation are mixed with 12 ml sample buffer (7 M urea, 2 M thiourea, 2% CHAPS, 0.4% IPG buffer pH 4-7, 0.5% DTT) and incubated for 1 h. The samples are concentrated in an Amicon^{®} Ultra-15 device (Millipore GmbH, Schwalbach, Germany) and the protein concentration is determined using the Bio-Rad^{®} protein assay (Cat.No. 500-0006; Bio-Rad Laboratories GmbH, München, Germany) following the instructions of the supplier's manual. To a volume corresponding to 1.5 mg of protein sample buffer is added to a final volume of 350 µl. This solution is used to rehydrate IPG strips pH 4-7 (Amersham Biosciences, Freiburg, Germany) overnight. The IEF is performed using the following gradient protocol: (1.) 1 minute to 500 V; (2.) 2 h to 3500 V; (3.) 22 h at constant 3500 V giving rise to 82 kVh. After IEF, strips are stored at -80°C or directly used for SDS-PAGE.

Prior to SDS-PAGE the strips are incubated in equilibration buffer (6 M urea, 50 mM Tris/HCl, pH 8.8, 30% glycerol, 2 % SDS), for reduction DTT (15 min, + 50 mg DTT/10 ml), and for alkylation IAA (15 min, + 235 mg iodacetamide/10 ml) is added. The strips are put on 12.5% polyacrylamide gels and subjected to electrophoresis at 1 W/gel and thereafter 1 h at 17 W/gel. Subsequently, the gels are fixed (50% methanol, 10% acetate) and stained overnight with Novex^{™} Colloidal Blue Staining Kit (Invitrogen, Karlsruhe, Germany, Cat No. LC6025, 45-7101)

### Detection of SPEE as a potential marker for breast cancer

Each patient is analyzed separately by image analysis with the ProteomeWeaver^{®} software (Definiens AG, Germany, München). In addition, all spots of the gel are excised by a picking robot and the proteins present in the spots are identified by MALDI-TOF mass spectrometry (Ultraflex^{™} Tof/Tof, Bruker Daltonik GmbH, Bremen, Germany). For each patient, 4 gels from the tumor sample are compared with 4 gels each from adjacent tissue and analyzed for distinctive spots corresponding to differentially expressed proteins. By this means, protein SPEE is found to be specifically expressed or strongly overexpressed in tumor tissue and not detectable in healthy control tissue. It therefore - amongst many other proteins - qualifies as a candidate marker for use in the diagnosis of breast cancer.

### Example 2

### Generation of antibodies to the breast cancer marker protein SPEE

Polyclonal antibody to the breast cancer marker protein SPEE is generated for further use of the antibody in the measurement of serum and plasma and blood levels of SPEE by immunodetection assays, e.g. Western Blotting and ELISA

### Recombinant protein expression and purification

In order to generate antibodies to SPEE, recombinant expression of the protein is performed for obtaining immunogens. The expression is done applying a combination of the RTS 100 expression system and E. coli. In a first step, the DNA sequence is analyzed and recommendations for high yield cDNA silent mutational variants and respective PCR-primer sequences are obtained using the "ProteoExpert RTS E.coli HY" system. This is a commercial web-based service (www.proteoexpert.com). Using the recommended primer pairs, the "RTS 100 E. coli Linear Template Generation Set, His-tag" (Roche Diagnostics GmbH, Mannheim, Germany, Cat.No. 3186237) system to generate linear PCR templates from the cDNA for in-vitro transcription and expression of the nucleotide sequence coding for the SPEE protein is used. For Western-blot detection and later purification, the expressed protein contains a His-tag. The best expressing variant is identified. All steps from PCR to expression and detection are carried out according to the instructions of the manufacturer. The respective PCR product, containing all necessary T7 regulatory regions (promoter, ribosomal binding site and T7 terminator) is cloned into the pBAD TOPO^{®} vector (Invitrogen, Karlsruhe, Germany, Cat. No. K 4300/01) following the manufacturer's instructions. For expression using the T7 regulatory sequences, the construct is transformed into E. coli BL 21 (DE 3) (Studier, F.W., et al., Methods Enzymol. 185 (1990) 60-89) and the transformed bacteria are cultivated in a 11 batch for protein expression.

Purification of His-SPEE fusion protein is done following standard procedures on a Ni-chelate column. Briefly, 11 of bacteria culture containing the expression vector for the His-SPEE fusion protein is pelleted by centrifugation. The cell pellet is resuspended in lysis buffer, containing phosphate, pH 8.0, 7 M guanidium chloride, imidazole and thioglycerole, followed by homogenization using a Ultra-Turrax^{®}. Insoluble material is pelleted by high speed centrifugation and the supernatant is applied to a Ni-chelate chromatographic column. The column is washed with several bed volumes of lysis buffer followed by washes with buffer, containing phosphate, pH 8.0 and urea. Finally, bound antigen is eluted using a phosphate buffer containing SDS under acid conditions.

### Synthesis of hemocyanin-peptide-conjugates for the generation of antibodies

Synthesis is carried out using heterobifunctional chemistry (maleimide/SH-chemistry). Selected cysteine containing SPEE-peptides are coupled to 3-maleimidohexanoyl-N-hydroxysuccinimidester (MHS) activated hemocyanin from Concholepas concholepas (Sigma, B-8556).

Hemocyanin is brought to 10 mg/ml in 100 mM NaH₂PO₄/NaOH, pH 7.2. Per ml hemocyanin 100 µl MHS (12.3 mg in DMSO) are added and incubated for 1 h. The sample is dialyzed over night against 100 mM NaH₂PO₄/NaOH, pH 6.5 and adjusted to 6 mg/ml with dialysis buffer. A selected cysteine containing SPEE-peptide was dissolved in DMSO (5 mg/ml for a peptide of 1500 Dalton). Per ml MHS-activated hemocyanin (6 mg/ml) 20 µl of 100 mM EDTA, pH 7.0 and 100 µl of the selected cysteine containing SPEE-peptide are added. After 1 h the remaining maleimide groups are blocked by the addition of 10 µl 0.5 M cysteine/HCl per ml reaction mixture. This preparation is used for immunization without further purification.

### Production of monoclonal antibodies against SPEE

### a) Immunization of mice

12 week old A/J mice are initially immunized intraperitoneally with 100 µg SPEE or hemocyanin-peptide-conjugate (see above). This is followed after 6 weeks by two further intraperitoneal immunizations at monthly intervals. In this process each mouse is administered 100 µg SPEE or hemocyanin-peptide-conjugate adsorbed to aluminium hydroxide and 10⁹ germs of *Bordetella pertussis.* Subsequently the last two immunizations are carried out intravenously on the 3rd and 2nd day before fusion using 100 µg SPEE or hemocyanin-peptide-conjugate in PBS buffer for each.

### b) Fusion and cloning

Spleen cells of the mice immunized according to a) are fused with myeloma cells according to Galfre, G., and Milstein, C., Methods in Enzymology 73 (1981) 3-46. In this process ca. 1x10⁸ spleen cells of the immunized mouse are mixed with 2x10⁷ myeloma cells (P3X63-Ag8-653, ATCC CRL1580) and centrifuged (10 min at 300 x g and 4°C.). The cells are then washed once with RPMI 1640 medium without foetal calf serum (FCS) and centrifuged again at 400 x g in a 50 ml conical tube. The supernatant is discarded, the cell sediment is gently loosened by tapping, 1 ml PEG (molecular weight 4000, Merck, Darmstadt) is added and mixed by pipetting. After 1 min in a water-bath at 37°C., 5 ml RPMI 1640 without FCS is added drop-wise at room temperature within a period of 4-5 min. Afterwards 5 ml RPMI 1640 containing 10% FCS is added drop-wise within ca. 1 min, mixed thoroughly, filled to 50 ml with medium (RPMI 1640+10% FCS) and subsequently centrifuged for 10 min at 400 x g and 4°C. The sedimented cells are taken up in RPMI 1640 medium containing 10% FCS and sown in hypoxanthine-azaserine selection medium (100 mmol/l hypoxanthine, 1 µg/ml azaserine in RPMI 1640+10% FCS). Interleukin 6 at 100 U/ml is added to the medium as a growth factor.

After ca. 10 days the primary cultures are tested for specific antibody. SPEE-positive primary cultures are cloned in 96 well cell culture plates by means of a fluorescence activated cell sorter. In this process again interleukin 6 at 100 U/ml is added to the medium as a growth additive.

### c) Immunoglobulin isolation from the cell culture supernatants

The hybridoma cells obtained are sown at a density of 1x10⁵ cells per ml in RPMI 1640 medium containing 10% FCS and proliferated for 7 days in a fermenter (Thermodux Co., Wertheim/Main, Model MCS-104XL, Order No. 144-050). On average concentrations of 100 µg monoclonal antibody per ml are obtained in the culture supernatant. Purification of this antibody from the culture supernatant is carried out by conventional methods in protein chemistry (e.g. according to Bruck, C., et al., Methods in Enzymology 121 (1986) 587-695).

### Generation of polyclonal antibodies

### a) Immunization

For immunization, a fresh emulsion of the protein solution (100 µg/ml SPEE or hemocyanin-peptide-conjugate) and complete Freund's adjuvant at the ratio of 1:1 is prepared. Each rabbit is immunized with 1 ml of the emulsion at days 1, 7,14 and 30, 60 and 90. Blood is drawn and resulting anti-SPEE serum used for further experiments as described in Examples 3 and 4.

### b) Purification of IgG (immunoglobulin G) from rabbit serum by sequential precipitation with caprylic acid and ammonium sulfate

One volume of rabbit serum is diluted with 4 volumes of acetate buffer (60 mM, pH 4.0). The pH is adjusted to 4.5 with 2 M Tris-base. Caprylic acid (25 µl/ml of diluted sample) is added drop-wise under vigorous stirring. After 30 min the sample is centrifuged (13,000 x g, 30 min, 4°C), the pellet discarded and the supernatant collected. The pH of the supernatant is adjusted to 7.5 by the addition of 2 M Tris-base and filtered (0.2 µm).

The immunoglobulin in the supernatant is precipitated under vigorous stirring by the drop-wise addition of a 4 M ammonium sulfate solution to a final concentration of 2 M. The precipitated immunoglobulins are collected by centrifugation (8,000 x g, 15 min, 4°C).

The supernatant is discarded. The pellet is dissolved in 10 mM NaH₂PO₄/NaOH, pH 7.5, 30 mM NaCl and exhaustively dialyzed. The dialysate is centrifuged (13,000 x g, 15 min, 4°C) and filtered (0.2 µm).

### Biotinylation of polyclonal rabbit IgG

Polyclonal rabbit IgG is brought to 10 mg/ml in 10 mM NaH₂PO₄/NaOH, pH 7.5, 30 mM NaCl. Per ml IgG solution 50 µl Biotin -N-hydroxysuccinimide (3.6 mg/ml in DMSO) are added. After 30 min at room temperature, the sample is chromatographed on Superdex 200 (10 mM NaH₂PO₄/NaOH, pH 7.5, 30 mM NaCl). The fraction containing biotinylated IgG are collected. Monoclonal antibodies are biotinylated according to the same procedure.

### Digoxygenylation of polyclonal rabbit IgG

Polyclonal rabbit IgG is brought to 10 mg/ml in 10 mM NaH₂PO₄/NaOH, 30 mM

NaCl, pH 7.5. Per ml IgG solution 50 µl digoxigenin-3-O-methylcarbonyl-ε-aminocaproic acid-N-hydroxysuccinimide ester (Roche Diagnostics, Mannheim, Germany, Cat. No. 1 333 054) (3.8 mg/ml in DMSO) are added. After 30 min at room temperature, the sample is chromatographed on Superdex^{®} 200 (10 mM NaH₂PO₄/NaOH, pH 7.5, 30 mM NaCl). The fractions containing digoxigenylated IgG are collected. Monoclonal antibodies are labeled with digoxigenin according to the same procedure.

### Example 3

### Western blot for the detection of SPEE in human serum and plasma samples.

SDS-PAGE and Western Blotting are carried out using reagents and equipment of Invitrogen, Karlsruhe, Germany. Human plasma samples are diluted 1:20 in reducing NuPAGE^{®} (Invitrogen) LDS sample buffer and heated for 5 min at 95°C. 10 µl aliquots are run on 4-12 % NuPAGE^{®} gels (Bis-Tris) in the MES running buffer system. The gel-separated protein mixture is blotted onto nitrocellulose membranes using the Invitrogen XCell II^{™} Blot Module (Invitrogen) and the NuPAGE^{®} transfer buffer system. The membranes are washed 3 times in PBS/0.05 % Tween-20 and blocked with SuperBlock Blocking Buffer (Pierce Biotechnology, Inc., Rockford, IL, USA). The biotinylated primary antibody is diluted in SuperBlock Blocking Buffer (0.01-0.2 µg/ml) and incubated with the membrane for 1h. The membranes are washed 3 times in PBS/0.05 % Tween-20. The specifically bound biotinylated primary antibody is labeled with a streptavidin-HRP-conjugate (20 mU_{ABTS}/ml in SuperBlock Blocking Buffer). After incubation for 1 h, the membranes are washed 3 times in PBS/0.05 % Tween-20. The bound streptavidin-HRP-conjugate is detected using a chemiluminescent substrate (SuperSignal West Femto Substrate, Pierce Biotechnology, Inc., Rockford, IL, USA) and autoradiographic film. Exposure times varies from 10 min to over night.

### Example 4

### ELISA for the measurement of SPEE in human serum and plasma samples.

For detection of SPEE in human serum or plasma, a sandwich ELISA is developed. For capture and detection of the antigen, aliquots of the anti-SPEE polyclonal antibody (see Example 2) are conjugated with biotin and digoxygenin, respectively.

Streptavidin-coated 96-well microtiter plates are incubated with 100 µl biotinylated anti-SPEE polyclonal antibody for 60 min at 10 µg/ml in 10 mM phosphate, pH 7.4, 1% BSA, 0.9% NaCl and 0.1% Tween-20. After incubation, plates are washed three times with 0.9% NaCl , 0.1% Tween-20. Wells are then incubated for 2 h with either a serial dilution of the recombinant protein (see Example 2) as standard antigen or with diluted plasma samples from patients. After binding of SPEE, plates are washed three times with 0.9% NaCl, 0.1% Tween-20. For specific detection of bound SPEE, wells are incubated with 100 µl of digoxygenylated anti-SPEE polyclonal antibody for 60 min at 10 µg/ml in 10 mM phosphate, pH 7.4, 1% BSA, 0.9% NaCl and 0.1% Tween-20. Thereafter, plates are washed three times to remove unbound antibody. In a next step, wells are incubated with 20 mU/ml anti-digoxigenin-POD conjugates (Roche Diagnostics GmbH, Mannheim, Germany, Catalog No. 1633716) for 60 min in 10 mM phosphate, pH 7.4, 1% BSA, 0.9% NaCl and 0.1% Tween-20. Plates are subsequently washed three times with the same buffer. For detection of antigen-antibody complexes, wells are incubated with 100 µl ABTS solution (Roche Diagnostics GmbH, Mannheim, Germany, Catalog No. 11685767) and OD is measured after 30-60 min at 405 nm with an ELISA reader.

### Example 5

### ROC analysis to assess clinical utility in terms of diagnostic accuracy.

Accuracy is assessed by analyzing individual liquid samples obtained from well-characterized patient cohorts, i.e., 50 patients having undergone mammography and found to be free of BC, 50 patients each diagnosed and staged as invasive ductal and invasive lobular T1-3, N0, M0 of BC, 50 patients diagnosed with progressed BC, having at least tumor infiltration in at least one proximal lymph node or more severe forms of metastasis, 50 patients each diagnosed with medullary, mucinous, tubular, or papillary breast carcinoma, and 50 patients diagnosed with DCIS, respectively. CA 15-3 as measured by a commercially available assay (Roche Diagnostics, CA 15-3-assay (Cat. No. 0 304 5838 for Elecsys^{®} Systems immunoassay analyzer) and SPEE measured as described above have been quantified in a serum obtained from each of these individuals. ROC-analysis is performed according to Zweig, M. H., and Campbell, *supra.* Discriminatory power for differentiating patients in the group Tᵢₛ-3, N0, M0 from healthy individuals for the combination of SPEE with the established marker CA 15-3 is calculated by regularized discriminant analysis (Friedman, J. H., Regularized Discriminant Analysis, Journal of the American Statistical Association 84 (1989) 165-175).

Preliminary data indicate that SPEE may also be very helpful in the follow-up of patients after surgery.

### List of References

Bruck, C., and Chen, G., et al., Methods Enzymol. 121 (1986) 587-695
Carney, P.A., et al., Ann. Intern. Med. 138 (2003) 168-175
Chen, G., et al., Molecular and Cellular Proteomics, 1.4 (2002) 304-313 Diamandis et al., eds. (1996) Immunoassay, Academic Press, Boston
Duffy, M.J., Critical Reviews in Clinical Laboratory Sciences 38 (2001) 225-262
Essermann, L., et al., J. Natl. Cancer Inst. 94 (2002) 369-375
Galfre, G., and Milstein, C., Methods Enzymol. 73 (1981) 3-46
Hibasami, H., et al., Anticancer Research 7 (1987) 1213-1216
Kaiser, A.E., et al., Folia Parasitologica 50 (2003) 3-18
Kuerer, H.M., et al., Cancer 95 (2002) 2276-2282
National Cancer Institute, Cancer Facts, Fact Sheet 5.18 (1998) 1-5
Nishikawa, Y., et al., Biochem. J. 321 (1997) 537-543
Nitta, T., et al., Exp. Cell Res. 276 (2002) 120-128
Singletary, S.E., et al. Journal of Clinical Oncology 20 (2002) 3628-3636
Studier, F.W., et al., Methods Enzymol. 185 (1990) 60-89
Tijssen, P., Practice and theory of enzyme immunoassays 11 (1990) the whole book, especially pages 43-78; Elsevier, Amsterdam
UICC (International Union Against Cancer), Sobin, L.H., Wittekind, Ch. (eds), -- TNM Classification of Malignant Tumours, fifth edition, 1997
Untch, M., et al., J. Lab. Med. 25 (2001) 343-352
Wahlfors, J., et al., DNA Cell Biol. 9 (1990) 103-110
WHO, Screening for Breast Cancer, May 10, 2002
WO 00/60076
WO 02/059377
WO 02/23200
Wulfkuhle, J.D., et al., Cancer Research 62 (2002) 6740-6749
Zweig, M. H., and Campbell, G., Clin. Chem. 39 (1993) 561-577

## Claims

1. A method for the diagnosis of breast cancer comprising the steps of
a) providing a liquid sample obtained from an individual,
b) contacting said sample with a specific binding agent for spermidine synthase (= SPEE) under conditions appropriate for formation of a complex between said binding agent and SPEE, and
c) correlating the amount of complex formed in (b) to the diagnosis of breast cancer.

2. The method according to claim 1, further **characterized in that** said sample is serum.

3. The method according to claim 1, further **characterized in that** said sample is plasma.

4. The method according to claim 1, further **characterized in that** said sample is whole blood.

5. The method according to claim 1, further **characterized in that** said sample is nipple aspirate fluid.

6. Use of protein SPEE as a marker molecule in the diagnosis of breast cancer from a liquid sample obtained from an individual.

7. Use of protein SPEE as a marker molecule in the early diagnosis of breast cancer from a liquid sample obtained from an individual.

8. Use according to claim 7, wherein the early diagnosis is made with a sample derived from BC patients in stage Tᵢₛ -3; N0; M0.

9. Use of protein SPEE as a marker molecule for breast cancer in combination with one or more marker molecules for breast cancer in the diagnosis of breast cancer from a liquid sample obtained from an individual.

10. Use according to claim 9, wherein the at least one other marker molecule is CA 15-3.

## Patentansprüche

1. Verfahren zur Diagnose von Brustkrebs, bei dem man die folgenden Schritte durchführt:
(a) Bereitstellen einer einem Individuum entnommenen Flüssigkeitsprobe,
(b) Inkontaktbringen der Probe mit einem spezifischen Bindungsmittel für Spermidinsynthase (= SPEE) unter zur Ausbildung eines Komplexes zwischen dem Bindungsmittel und SPEE geeigneten Bedingungen und
(c) Korrelieren der Menge an in (b) gebildetem Komplex mit der Diagnose von Brustkrebs.

2. Verfahren nach Anspruch 1, ferner **dadurch gekennzeichnet, daß** es sich bei der Probe um Serum handelt.

3. Verfahren nach Anspruch 1, ferner **dadurch gekennzeichnet, daß** es sich bei der Probe um Plasma handelt.

4. Verfahren nach Anspruch 1, ferner **dadurch gekennzeichnet, daß** es sich bei der Probe um Vollblut handelt.

5. Verfahren nach Anspruch 1, ferner **dadurch gekennzeichnet, daß** es sich bei der Probe um Aspirat aus der Brustwarze handelt.

6. Verwendung des Proteins SPEE als ein Markermolekül bei der Diagnose von Brustkrebs aus einer einem Individuum entnommenen Flüssigkeitsprobe.

7. Verwendung des Proteins SPEE als ein Markermolekül bei der Frühdiagnose von Brustkrebs aus einer einem Individuum entnommenen Flüssigkeitsprobe.

8. Verwendung nach Anspruch 7, wobei die Frühdiagnose an einer von Brustkrebspatienten im Stadium Tᵢₛ -3; N0; M0 stammenden Probe vorgenommen wird.

9. Verwendung des Proteins SPEE als ein Markermolekül für Brustkrebs in Kombination mit einem oder mehreren Markermolekülen für Brustkrebs bei der Diagnose von Brustkrebs aus einer einem Individuum entnommenen Flüssigkeitsprobe.

10. Verwendung nach Anspruch 9, wobei das mindestens eine andere Markermolekül CA 15-3 ist.

## Revendications

1. Procédé de diagnostic du cancer du sein comprenant les étapes de
a) fourniture d'un échantillon de liquide prélevé sur un individu,
b) mise en contact dudit échantillon avec un agent de liaison spécifique de la spermidine synthase (=SPEE) dans des conditions appropriées pour la formation d'un complexe entre ledit agent de liaison et la SPEE, et
c) corrélation de la quantité de complexe formé en (b) au diagnostic de cancer du sein.

2. Procédé selon la revendication 1, **caractérisé en outre en ce que** ledit échantillon est du sérum.

3. Procédé selon la revendication 1, **caractérisé en outre en ce que** ledit échantillon est du plasma.

4. Procédé selon la revendication 1, **caractérisé en outre en ce que** ledit échantillon est du sang total.

5. Procédé selon la revendication 1, **caractérisé en outre en ce que** ledit échantillon est du liquide d'aspiration mamelonnaire.

6. Utilisation de la protéine SPEE comme molécule marqueur dans le diagnostic du cancer du sein à partir d'un échantillon de liquide prélevé sur un individu.

7. Utilisation de la protéine SPEE comme molécule marqueur dans le diagnostic précoce du cancer du sein à partir d'un échantillon de liquide prélevé sur un individu.

8. Utilisation selon la revendication 7, d ans laquelle le diagnostic précoce est réalisé avec un échantillon dérivé de patients ayant un CS au stade Tᵢₛ -3 ; NO ; M0.

9. Utilisation de la protéine SPEE comme molécule marqueur pour le cancer du sein en combinaison avec une ou plusieurs molécules marqueurs du cancer du sein dans le diagnostic du cancer du sein à partir d'un échantillon de liquide prélevé sur un individu.

10. Utilisation selon la revendication 9, dans laquelle la au moins une autre molécule marqueur est le CA 15-3.
